# EUROPEAN PATENT APPLICATION

(11) **EP 3 483 256 A1**
(43) Date of publication of application: **15.05.2019**
(21) Application number: 17827905.5
(22) Date of filing: 11.07.2017
(51) Int. Cl.: C12N 1/20, A61K 35/66, A61K 35/74, A23L 33/135, A23K 10/16, C12R 1/01

(54) **AKKERMANSIA MUCINIPHILA STRAIN HAVING EFFECT OF PREVENTING OR TREATING DEGENERATIVE BRAIN DISEASES OR METABOLIC DISEASES, AND USE THEREOF**

(30) Priority: 11.07.2016 KR 20160087473; 11.07.2016 KR 20160087474
(71) Applicant: Korea Research Institute of Bioscience and Biotechnology, Daejeon 34141 (KR)
(72) Inventor: KIM, Byoung-Chan, Daejeon 34141 (KR); LEE, Chul-Ho, Daejeon 34141 (KR); NOH, Jung-Ran, Daejeon 34141 (KR); KIM, Kyoung-Shim, Daejeon 34141 (KR); KIM, Myung-Hee, Daejeon 34141 (KR); KIM, Yong-Hoon, Daejeon 34141 (KR); LEE, Sang Jun, Daejeon 34141 (KR); CHANG, Dong-Ho, Daejeon 34141 (KR); CHOI, Dong-Hee, Daejeon 34141 (KR); HWANG, Jung Hwan, Daejeon 34141 (KR); SEO, Yun-Jung, Daejeon 34141 (KR); LEE, In-Bok, Daejeon 34141 (KR); CHOI, Young-Keun, Daejeon 34141 (KR); CHOI, Jung-Hyeon, Daejeon 34141 (KR)
(74) Representative: Sonn & Partner Patentanwälte
(86) International application number: PCT/KR2017/007370
(87) International publication number: WO 2018/012834

(57) **Abstract**

The present disclosure relates to an *Akkermansia muciniphila* strain having a prophylactic or therapeutic effect on a degenerative brain disease and uses thereof. Since the *Akkermansia muciniphila* strain, the intestinal microorganism of the present disclosure, shows an effect of improving movement control and cognitive abilities as well as memory in an animal model having a degenerative brain disease such as Parkinson's disease and Alzheimer's disease, it can be useful in the prevention or treatment of brain diseases including Alzheimer's disease, Parkinson's disease, mild cognitive impairment, *etc.* In addition, it was confirmed in the present disclosure that compared to the *Akkermansia muciniphila* strain cultured in a mucin-containing medium, that cultured in a mucin-free medium showed a remarkable effect of improving hyperlipidemia, fatty liver, obesity, and hyperglycemia induced in a mouse model by high-fat diet when administered orally (*in vivo*). Accordingly, the present disclosure is expected to be very useful in relevant industries as a particular composition of culture medium and an optimized obligatory anaerobic culture method have been developed through the present disclosure.

## Description

### Technical Field

The present disclosure relates to an *Akkermansia muciniphila* strain having a prophylactic or therapeutic effect on a degenerative brain disease or metabolic disease and use thereof.

### Background Art

Memory is becoming increasingly important in the rapidly changing lives of modern people and from socially educated adolescents to the elderly, it is of major interest. Degenerative brain diseases are known to lead to memory decline, and brain inflammation has been revealed to be an important factor in the degenerative brain diseases of the central nervous system, such as Alzheimer's syndrome, Parkinson's syndrome, and Huntington's syndrome. Recently, as the number of patients with a degenerative brain disease such as dementia has been rapidly increased due to an increase in the elderly population, there have been efforts made to develop various therapeutic strategies to improve and enhance the cognitive and learning functions that have deteriorated due to dementia and to develop effective drugs. The drugs that have thus far been developed for the improvement of memory are acetylcholine precursors, receptor agonists, acetylcholine esterase inhibitor, *etc.* However, there have not yet been any therapeutic drugs developed which are capable of treating the fundamental pathogenesis of degenerative brain diseases, although there are conventional therapeutic agents available such as acetylcholinesterase inhibitors (*e.g.,* Aricept (Pfizer), Excelon (Novartis), and Reminyl (Yansen)) and agonists of *N*-methyl-D-aspartate receptor (NMDA) that have recently been approved by the US FDA (*e.g.,* Ebixa (Memantine: Lundbeck). However, the acetylcholinesterase inhibitor can merely improve cognitive ability that has declined, but is limited in that it cannot treat the fundamental onset of Alzheimer's disease. In addition, it is known that it is difficult to expect a fundamental therapeutic effect as only some patients temporarily show a symptom-mitigation effect, and its medicinal effect does not last long. Due to the characteristics of degenerative brain diseases, long-term administration of medicine is required; however, in the case of the above drugs, there are problems such as some accompanying side effects such as hepatotoxicity, vomiting, and loss of appetite. Therefore, there is an urgent need to develop new therapeutic drugs that can prevent the progressive process of degenerative brain diseases. To this end, many multinational pharmaceutical companies have been making huge investments in research and development in this field, and the development of beta or gamma secretase inhibitors which reduce the production of β-amyloid consisting of about 40 amino acids, which are suspected of being a fundamental pathogen of Alzheimer's disease, accounts for the largest part thereof. Domestically, basic research on Alzheimer's disease has been done to some extent, but there has not been any development of a therapeutic agent itself for dementia.

Meanwhile, metabolic diseases comprehensively refer to diseases caused by metabolic disorders *in vivo,* and are generally caused by an imbalance of carbohydrates, lipids, proteins, vitamins, electrolytes, water, *etc.* Obesity, diabetes mellitus, hyperlipidemia, arteriosclerosis, hypertension, *etc.* are examples thereof. Among these, diabetes mellitus type 2 is a type of diabetes which mainly develops in adults and is characterized by an increased resistance to insulin. Sensitivity to insulin decreases when the number of insulin receptors is reduced or the sensitivity of the receptors decreases, or when there is a problem in the second transporter which causes glycogen synthesis in cells. Accordingly, type 2 diabetes is called non-insulin-independent diabetes and accounts for 85% to 90% of diabetes. In particular, obesity is a social problem for aesthetic reasons; however, the most serious problem associated with obesity is that it can result in serious health risks such as complications of diabetes, hypertension, and other metabolic disorders. A symptom related to the pathological condition of obesity is systemic chronic inflammation appearing in obese subjects. Inflammatory reactions, as one of the immune mechanisms occurring in the body, are important in protecting the body from invasion of pathogenic bacteria and viruses from the outside when they occur locally. However, when such inflammatory reactions are hyperactivated systemically and chronically due to an imbalance of the immune reactions in the body, they cause disorders in metabolic actions occurring in the body. The chronic inflammatory responses induced by obesity have been identified as a cause of various metabolic diseases such as diabetes, cardiovascular diseases, arteriosclerosis, *etc.,* and are also the most important factor in regulating obesity as a disease. Obesity without the onset of secondary metabolic diseases caused by chronic inflammatory reactions is simply a cosmetic problem, and has recently been established by the World Health Organization as a disease due to chronic inflammatory reactions capable of causing secondary metabolic diseases that significantly diminish the quality of life. Currently, a steady increase in the intake of foods with high energy load and social changes associated with less movement have increased the incidence of obesity and metabolic diseases caused thereby. Traditional treatment based on low-calorie diet and exercise does not show much effect in controlling obesity, and only leads to temporary weight loss. Development of a safe and effective medication that induces weight loss has been underway for decades. To date, however, drugs that have shown efficacy have serious side effects or have not been very effective in clinical trials. Therefore, there is a need for a new approach that can improve obesity and metabolic diseases caused by obesity and prevent these pathological conditions.

The results of recent studies have revealed that intestinal microorganisms positively influence human intestinal health and bowel disorders, as well as regulation of brain functions and development of brain diseases. However, the prophylactic or therapeutic effects of *Akkermansia muciniphila* strain on the brain diseases and on brain cognition memory have not yet been investigated, and effective prophylactic or therapeutic effects of the strain cultured under specific culture conditions have not been known.

Meanwhile, KR Patent No. 1476236 discloses *"Lactobacillus* having activity of preventing and/or treating aging and dementia", and Korean Laid-open Application No. 2015-0093711 discloses "use of *Akkermansia* for treating metabolic disorders". In contrast to the present disclosure, however, said patent and application are silent on *Akkermansia muciniphila* strain having a prophylactic or therapeutic effect on a degenerative brain disease or metabolic disease and use thereof.

### Disclosure

### Technical Problem

The inventors of the present disclosure, which was derived as a result of the demand, endeavored to develop as a safe drug capable of effectively inhibiting degenerative brain diseases such as Parkinson's disease and Alzheimer's disease, for which the risk of development is increasing, and of treating without any side effects a prophylactic or therapeutic agent for degenerative brain diseases using a substance which is not toxic to the human body upon intake, and as a result, they found that *Akkermansia muciniphila* (AK : Accession No. ATCC BAA-835, identical to the strain of German Collection of Microorganisms and Cell Cultures Accession No. DSMZ 22959), the intestinal strain already known as a standard strain, has an effect on the diseases.

Additionally, the inventors of the present disclosure endeavored to develop as a safe drug capable of effectively inhibiting metabolic brain diseases such as hyperlipidemia, fatty liver, obesity, and diabetes and treating without any side effects a prophylactic or therapeutic agent for degenerative brain diseases using a substance which is not toxic to the human body upon intake, and as a result, they found that *Akkermansia muciniphila* (AK : Accession No. ATCC BAA-835, identical to the strain of German Collection of Microorganisms and Cell Cultures Accession No. DSMZ 22959), the intestinal strain already known as a standard strain, has an effect on the diseases.

In particular, the inventors of the present disclosure revealed that the *Akkermansia muciniphila* strain had improved effects on movement disorder in an animal model with Parkinson's disease and cognitive ability and memory in an animal model with Alzheimer's disease, when administered orally (*in vivo*), and thus can be used for the prevention or treatment of degenerative brain diseases and improvement of brain cognition and memory. The inventors have also revealed that the *Akkermansia muciniphila* strain cultured in a mucin-free medium had greater improvement effects on hyperlipidemia, fatty liver, obesity, and hyperglycemia compared to that cultured in a mucin-containing medium, when administered orally (*in vivo*), thereby completing the present disclosure.

### Technical Solution

In order to solve the above problems, the present disclosure provides an *Akkermansia muciniphila* strain having a prophylactic or therapeutic effect on a degenerative brain disease.

Additionally, the present disclosure provides a pharmaceutical composition for preventing or treating a degenerative brain disease, comprising as an active ingredient at least one selected from the group consisting of the strain, an endoplasmic reticulum derived therefrom, a culture thereof, a concentrate of the culture, a dry matter of the culture, and an extract of the culture.

Additionally, the present disclosure provides a pharmaceutical composition for preventing or treating a degenerative brain disease, comprising as an active ingredient at least one selected from the group consisting of the strain, an endoplasmic reticulum derived therefrom, a culture thereof, a concentrate of the culture, a dry matter of the culture, and an extract of the culture.

Additionally, the present disclosure provides a health functional food composition for preventing or improving a degenerative brain disease, comprising as an active ingredient at least one selected from the group consisting of the strain, an endoplasmic reticulum derived therefrom, a culture thereof, a concentrate of the culture, a dry matter of the culture, and an extract of the culture.

Additionally, the present disclosure provides a feed additive composition for preventing or improving a degenerative brain disease, comprising as an active ingredient at least one selected from the group consisting of the strain, an endoplasmic reticulum derived therefrom, a culture thereof, a concentrate of the culture, a dry matter of the culture, and an extract of the culture.

Additionally, the present disclosure provides a pharmaceutical composition for preventing or treating a metabolic disease, comprising as an active ingredient at least one selected from the group consisting of an *Akkermansia muciniphila* strain cultured in a mucin-free medium, an endoplasmic reticulum derived therefrom, a culture thereof, a concentrate of the culture, a dry matter of the culture, and an extract of the culture.

Additionally, the present disclosure provides a health functional food composition for preventing or treating a metabolic disease, comprising as an active ingredient at least one selected from the group consisting of an *Akkermansia muciniphila* strain cultured in a mucin-free medium, an endoplasmic reticulum derived therefrom, a culture thereof, a concentrate of the culture, a dry matter of the culture, and an extract of the culture.

Additionally, the present disclosure provides a feed additive composition for preventing or treating a metabolic disease, comprising as an active ingredient at least one selected from the group consisting of an *Akkermansia muciniphila* strain cultured in a mucin-free medium, an endoplasmic reticulum derived therefrom, a culture thereof, a concentrate of the culture, a dry matter of the culture, and an extract of the culture.

### Advantageous Effects

Since the *Akkermansia muciniphila* strain, the intestinal microorganism of the present disclosure, shows an effect of improving movement control and cognitive abilities as well as memory in an animal model having a degenerative brain disease such as Parkinson's disease and Alzheimer's disease, it can be useful in the prevention or treatment of brain diseases including Alzheimer's disease, Parkinson's disease, mild cognitive impairment, *etc.* In addition, it was confirmed in the present disclosure that compared to the *Akkermansia muciniphila* strain cultured in a mucin-containing medium, that cultured in a mucin-free medium showed a remarkable effect of improving hyperlipidemia, fatty liver, obesity, and hyperglycemia induced in a mouse model by high-fat diet when administered orally (*in vivo*). Accordingly, the present disclosure is expected to be very useful in relevant industries, as a particular composition of culture medium and an optimized obligatory anaerobic culture method have been developed through the present disclosure.

### Brief Description of Drawings

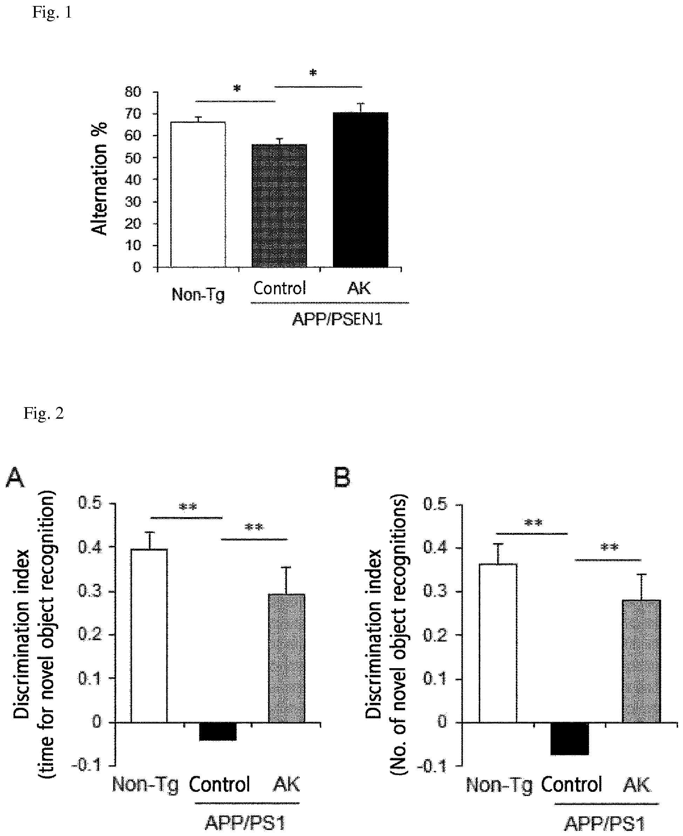

Fig. 1 is a graph showing effects of improving space perception ability and alleviating short-term memory failure during a Y maze test by administering the *Akkermansia muciniphila* (AK), the intestinal microorganism according to the present disclosure, to a mouse (B6C3-Tg(APPswe/PSEN1dE9)85DboJ, JAX, 004462) having Alzheimer's diseases due to overexpressed Alzheimer's disease-related APP and PSEN1 genes in the brain.
Fig. 2 is graphs showing improvement effects of cognitive ability and memory during a novel object recognition test (NORT; A: time for novel object recognition; B: number of novel object recognitions) by administering the *Akkermansia muciniphila* (AK), the intestinal microorganism according to the present disclosure, to a mouse (B6C3-Tg(APPswe/PSEN1dE9)85DboJ, JAX, 004462) having Alzheimer's diseases due to overexpressed Alzheimer's disease-related APP and PSEN1 genes in the brain.

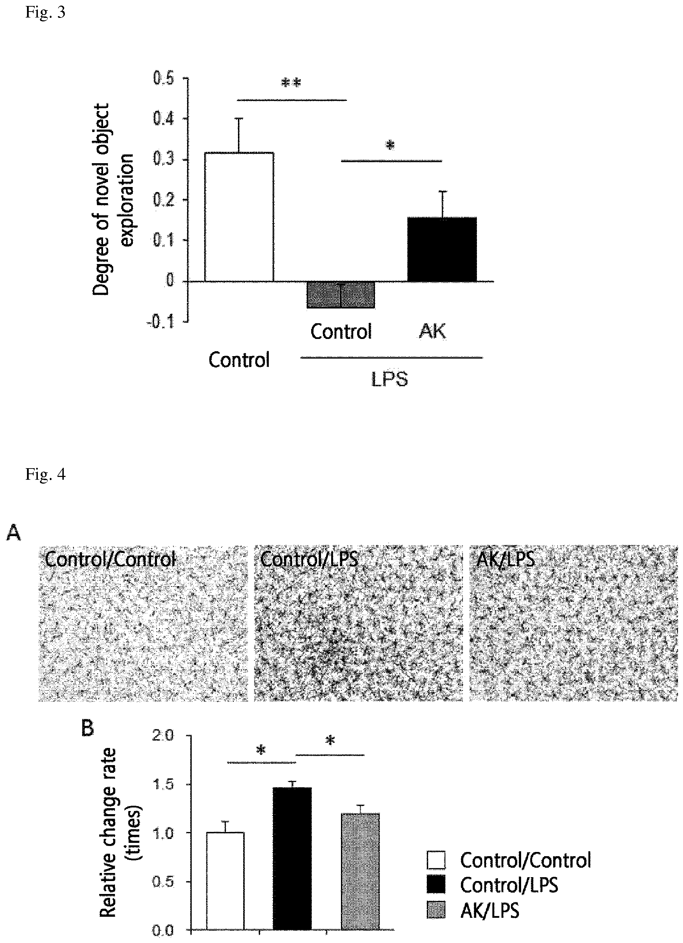

Fig. 3 is a graph showing improvement effects of cognitive ability and memory during a novel object recognition test (NORT) by administering the *Akkermansia muciniphila* (AK), the intestinal microorganism according to the present disclosure, to a mouse having cognitive function damage induced by LPS administration.
Fig. 4 is photos (A) and a graph (B) showing the results of immunostaining for microneuroglial cells hyperactivated in the brain of a mouse having cognitive function damaged by LPS administration by administering the *Akkermansia muciniphila* strain, the intestinal microorganism according to the present disclosure.

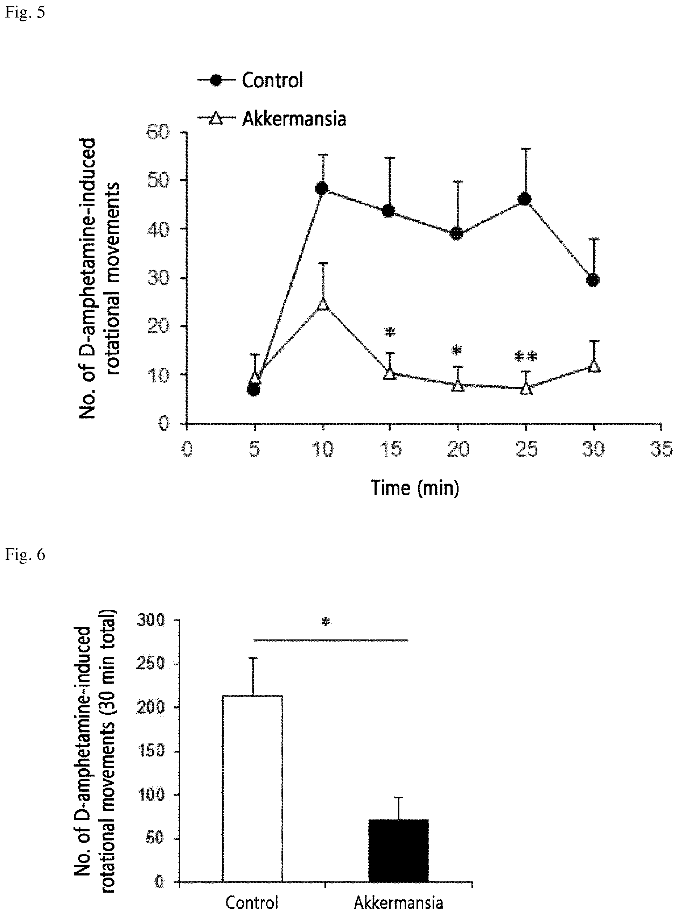

Fig. 5 is a graph showing the reduction of the number of D-amphetamine-induced rotational movements according to the treatment of the intestinal microorganism *Akkermansia muciniphila* strain in a mouse model having Parkinson's disease, in which dopaminergic neuron-specific cell death is caused by 6-OHDA measured every 5 minutes.
Fig. 6 is a graph showing the sum of the numbers of D-amphetamine-induced rotational movements according to the treatment of the intestinal microorganism *Akkermansia muciniphila* strain in a mouse model having Parkinson's disease, in which dopaminergic neuron-specific cell death is caused by 6-OHDA measured for 30 minutes, which is decreased compared to the control.

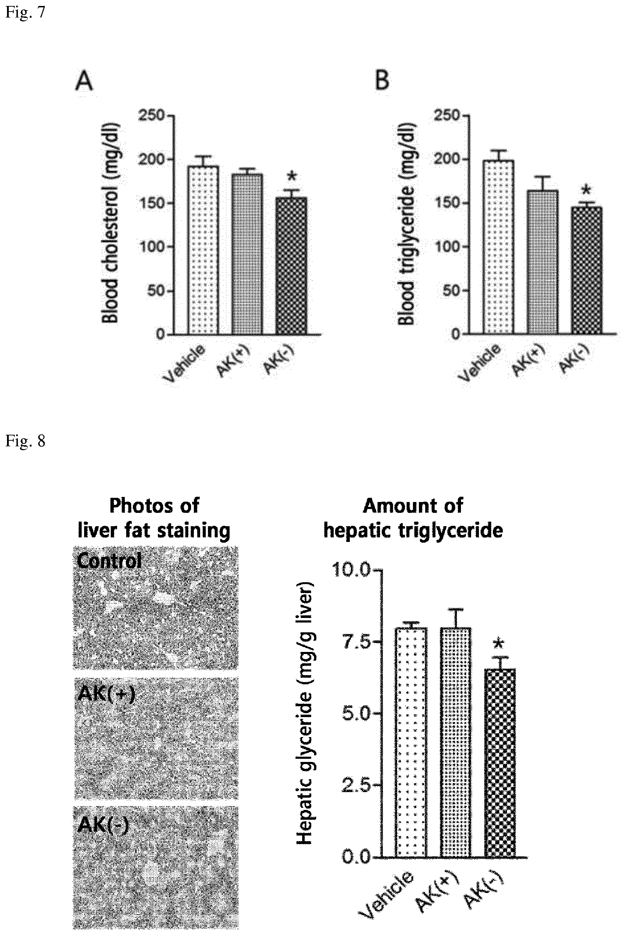

Fig. 7 shows the hyperlipidemia inhibition effect by AK(+) and AK(-) administration to mice models having hyperlipidemia induced by high-fat diet, represented by the changes in the blood cholesterol contents (A) and those in the blood triglyceride contents (B). **p*<0.01 *vs.* experimental control group (student's *t*-test).
Fig. 8 shows the effect of fatty liver inhibition by AK(+) and AK(-) administration to mice models having fatty liver induced by high-fat diet, confirmed by fatty liver staining (left) and represented by the changes in the blood triglyceride contents (B). **p*<0.01 *vs.* experimental control group (student's *t*-test).

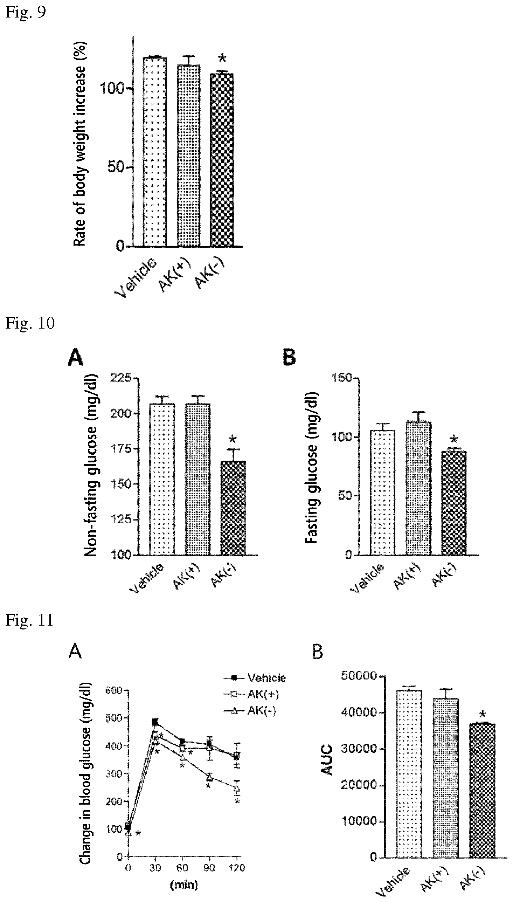

Fig. 9 shows the result of comparison of rates of body weight increase of mice models having fatty liver induced by high-fat diet between administration of AK(+) and AK(-) strains. **p*<0.01 *vs.* experimental control group (student's *t*-test)
Fig. 10 shows the reduced blood glucose effect by AK(+) and AK(-) administration to mice models having hyperglycemia induced by high-fat diet, represented by the changes in the non-fasting glucose (A) and those in the fasting glucose (B). **p*<0.01 *vs.* experimental control group (student's *t*-test).
Fig. 11 shows the reduced blood glucose effect by AK(+) and AK(-) administration to mice models having impaired glucose tolerance induced by high-fat diet, represented by the changes in the blood glucose (A) and AUC (B). AUC: area under curve, **p*<0.01 *vs.* experimental control group (student's *t*-test).

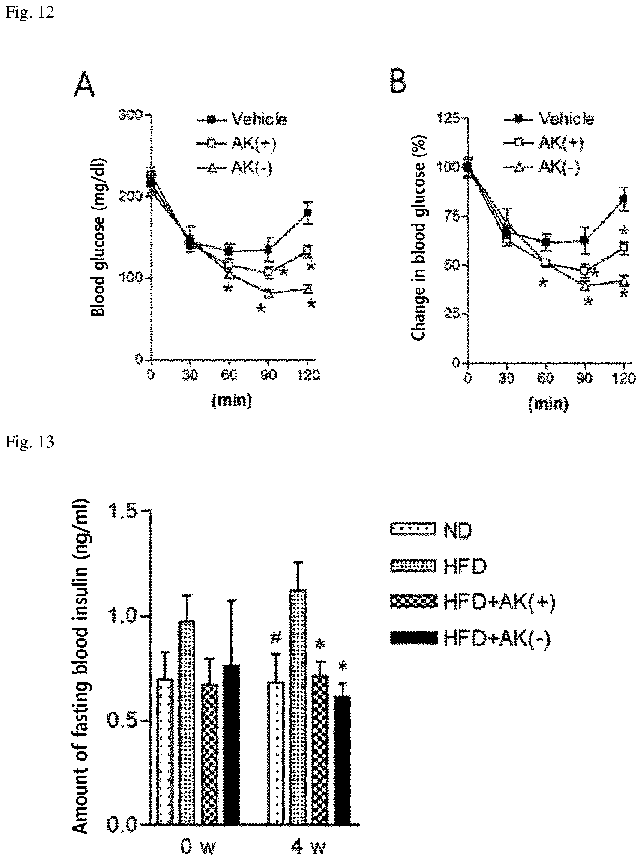

Fig. 12 shows the effect of enhanced glucose tolerance by AK(+) and AK(-) administration to mice models having reduced sensitivity to insulin induced by high-fat diet, measured by the changes in the blood glucose (A) and the blood glucose change rate (B). **p*<0.01 *vs*. experimental control group (student's *t*-test).
Fig. 13 shows the effect of enhanced sensitivity to insulin by AK(+) and AK(-) administration to mice models having reduced sensitivity to insulin induced by high-fat diet, represented by the insulin concentration in blood. ND: control group with normal diet; no strain-treated, HFD: experimental group with high-fat diet; no strain-treated, HFD+AK(+): experimental group proliferated in mucin-containing medium; administered with AK(+) strain, HFD+AK(-): experimental group proliferated in mucin-free medium; administered with AK(-) strain. **p*<0.01 *vs.* experimental control group (student's *t*-test).

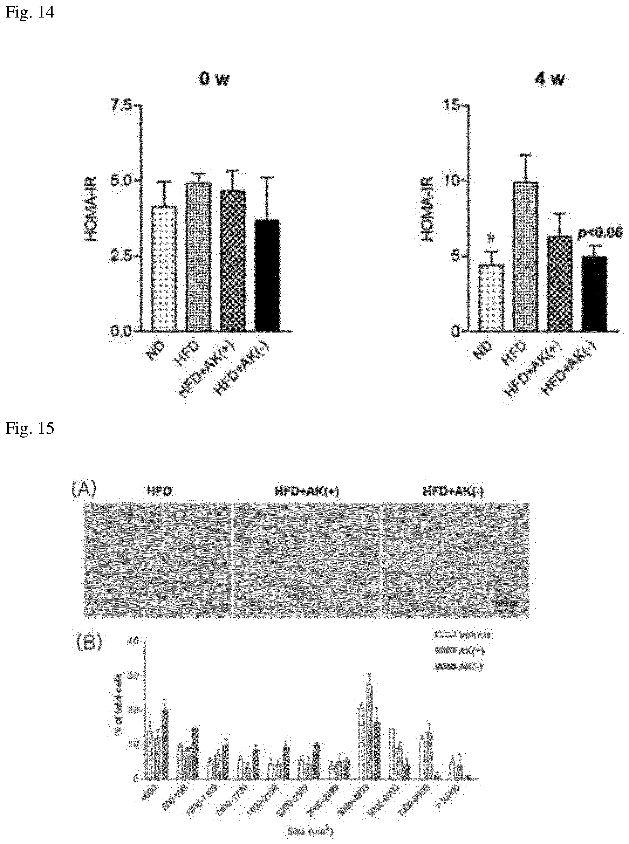

Fig. 14 shows the effects of enhanced glucose tolerance and sensitivity to insulin by AK(+) and AK(-) administration to mice models having reduced sensitivity to insulin induced by high-fat diet, represented by calculation of Homeostasis Model Assessment of insulin resistance index (HOMA-IR, [Fasting insulin (µIU/mL) × fasting plasma glucose (mmol/L)] / 22.5), generally used as an index for insulin resistance. ND: control group with normal diet; no strain-treated, HFD: experimental group with high-fat diet; no strain-treated, HFD+AK(+): experimental group proliferated in mucin-containing medium; administered with AK(+) strain, HFD+AK(-): experimental group proliferated in mucin-free medium; administered with AK(-) strain. **p*<0.01 *vs.* experimental control group (student's *t*-test).
Fig. 15 shows the results of photo-taking (A) the changes in gonadal fat caused by AK(+) and AK(-) administration and measuring the size (B) thereof in mice models having obesity induced by high-fat diet so as to confirm that AK(-) strain effectively inhibits adipocyte proliferation that is caused by high-fat diet. HFD: experimental group with high-fat diet; no strain-treated, HFD+AK(+): experimental group proliferated in mucin-containing medium; administered with AK(+) strain, HFD+AK(-): experimental group proliferated in mucin-free medium; administered with AK(-) strain.

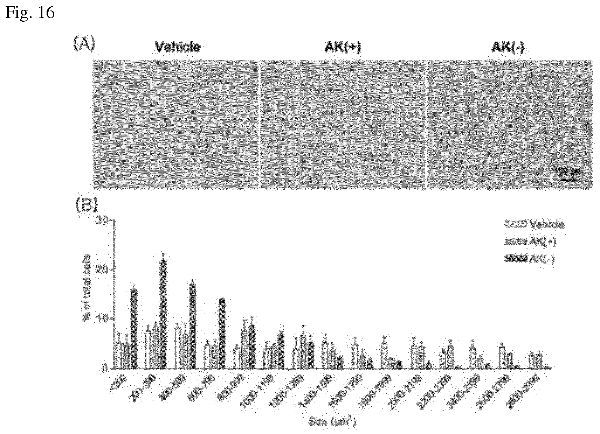

Fig. 16 shows the results of photo-taking (A) the changes in inguinal fat caused by AK(+) and AK(-) administration and measuring the size (B) thereof in mice models having obesity induced by high-fat diet so as to confirm that AK(-) strain effectively inhibits adipocyte proliferation that is caused by high-fat diet. Vehicle: vehicle-treated experimental group with high-fat diet, AK(+): experimental group proliferated in mucin-containing medium; administered with AK(+) strain, AK(-): experimental group proliferated in mucin-free medium; administered with AK(-).

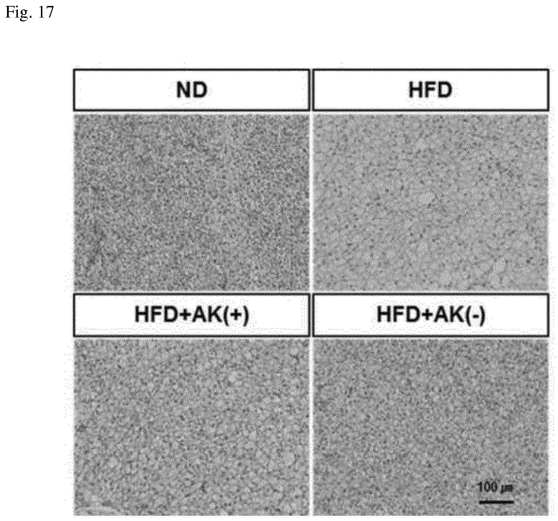

Fig. 17 shows the results of H&E staining of the changes in the brown fat by AK(+) and AK(-) administration in mice models having obesity induced by high-fat diet, confirming that AK(-)strain effectively inhibits reduction of brown fat by high-fat diet and maintain the reduction and maintains brown fat similarly to normal mice. ND: control group with normal diet; no strain-treated, HFD: experimental group with high-fat diet; no strain-treated, HFD+AK(+): experimental group proliferated in mucin-containing medium; administered with AK(+) strain, HFD+AK(-): experimental group proliferated in mucin-free medium; administered with AK(-) strain.

### Detailed Description of the Invention

In order to achieve the objects, the present disclosure provides an *Akkermansia muciniphila* strain having a prophylactic or therapeutic effect on a degenerative brain disease.

Currently, there has been no disclosure relevant to use of the *Akkermansia muciniphila* strain on the prevention or treatment of degenerative brain diseases, *etc.,* and it was first investigated by the present inventors that the strain is used to prevent or treat degenerative brain diseases including Alzheimer's disease, Parkinson's disease, *etc.* According to existing journal report (Pharmacology & Therapeutics 2016. 158: 52-62), intestinal microorganisms of patients with dementia such as Alzheimer's disease or Parkinson's disease are different from those of healthy individuals. However, the first patent or journal article which first substantially proved the prophylactic or therapeutic effect of the intestinal microorganisms of healthy individuals on dementia such as Alzheimer's disease, Parkinson's disease, *etc.* is the present disclosure, where possibility to develop a human symbiotic microorganism as a prophylactic or therapeutic drug for dementia was first revealed worldwide.

The *Akkermansia muciniphila* strain of the present disclosure was furnished from American Type Culture Collection (ATCC) to use in the present disclosure (Accession No. ATCC BAA-835, the strain identical to German Collection of Microorganisms and Cell Cultures Accession No. DSMZ 22959).

As used herein, the term "degenerative brain disease" refers to one of the degenerative diseases that develop in the brain with advancing years and preferably one selected from the group consisting of Alzheimer's disease, Parkinson's disease, mild cognitive disorder, meningitis, palsy, dementia, Huntington's disease, Creutzfeldt-Jakob disease, and combinations thereof, but is not limited thereto. It is known that protein aggregation due to neurodegeneration and genetic and environmental factors causes neuronal cell death, which will lead to degenerative brain diseases; however, the exact cause thereof has not yet been revealed.

As used herein, the term "prevention" refers to all behaviors involved in inhibition or delay of the development of degenerative brain disease by administration of the pharmaceutical composition of the present disclosure, and the term "treatment" refers to all behaviors resulting in alleviation of symptoms of said disease or beneficial alteration thereof by administering the pharmaceutical composition.

The prevention or treatment of the degenerative brain diseases can be obtained by the microorganism strain and by having inhibitory effects against phosphorylation of amyloid precursor protein and against brain inflammatory action. Ethologically, the prevention or treatment effect can be achieved by improvement of movement control disability as well as of cognitive ability and memory in animal models with degenerative brain diseases such as Parkinson's disease and Alzheimer's disease.

In addition, the present disclosure provides a pharmaceutical composition for preventing or treating a degenerative brain disease, comprising as an active ingredient at least one selected from the group consisting of the strain, an endoplasmic reticulum derived therefrom, a culture thereof, a concentrate of the culture, a dry matter of the culture, and an extract of the culture.

In the pharmaceutical composition according to an exemplary embodiment of the present disclosure for preventing or treating a degenerative brain disease, the composition may have an inhibitory effect against movement disorder caused by dopaminergic neuron death or neuroinflammation or an effect of improving cognitive ability and memory, but is not limited thereto.

As used herein, the term "dopamine" refers to a neurotransmitter that transmits a signal in the brain and is known to be relevant to movement and motility.

As used herein, the term "dopaminergic neuron death" refers to loss or degeneration of dopaminergic neurons concentrated in the *substantia nigra pars compacta,* and it is known that an animal model of Parkinson's disease having dopaminergic neuron death induced can be prepared by injecting 6-hydroxyldopamine (6-OHDA).

As used herein, the term "neuroinflammation" is an important factor that causes degenerative brain diseases. It is known that when inflammatory cells are hyper-activated, secretion of pro-inflammatory cytokine increases, and hyperactivation of such brain inflammatory reaction will induce degenerative brain diseases such as brain cell loss, Parkinson's disease, and Alzheimer's disease.

As previously described, the *Akkermansia muciniphila* strain provided in the present disclosure was confirmed to have an inhibitory effect against brain inflammatory reaction. Further, the strain can improve movement-controlling ability, cognitive ability, and memory in an animal model having a degenerative brain disease such as Parkinson's disease and Alzheimer's disease.

According to an exemplary embodiment, it was confirmed in the present disclosure that statistically, alternation significantly improved in animal models with cognitive impairment and Alzheimer type dementia (Fig. 1), and that statistically, time for novel object recognition significantly increased (Figs. 2 and 3). Further, an animal model having Parkinson's disease, in which 6-OHDA was used to induce dopaminergic neuron-specific death in the brain, was observed to analyze its dextroamphetamine-induced rotational behavior, and as a result showed significantly decreased rotational behavior due to the microorganism strain (Figs. 5 and 6).

Accordingly, the gut microorganism *Akkermansia muciniphila* strain was confirmed to have effects of improving movement-controlling ability, cognitive ability, and memory in animal models having a degenerative brain disease such as Parkinson's disease and Alzheimer's disease.

The pharmaceutical composition of the present disclosure may further comprise a carrier, excipient, and diluent conventionally used in the preparation of a pharmaceutical composition.

The pharmaceutical composition according to the present disclosure may be prepared into an oral formulation such as a powder, granule, tablet, capsule, suspension, emulsion, syrup, or aerosol, an external formulation, a suppository formulation, and a sterilized injection solution formation, according to conventional methods. The carriers, excipient, and diluents that can be contained in the pharmaceutical composition according to the present disclosure may be lactose, dextrose, sucrose, sorbitol, mannitol, xylitol, erythritol, maltitol, starch, Acacia rubber, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oils, and other various compounds or mixtures. The pharmaceutical composition is prepared into a formulation using a commonly used diluent or excipient such as a filler, extender, binding agent, wetting agent, disintegrant, surfactant, *etc.* Solid formulations for oral administration may include a tablet, pill, powder, granule, capsule, *etc.,* and are prepared by mixing at least one excipient, *e.g.,* starch, calcium carbonate, sucrose, lactose, gelatin, *etc.* in the strain or an endoplasmic reticulum derived therefrom. In addition to the simple excipient, a lubricant such as magnesium stearate and talc may also be used. Liquid formulations for oral administration may include a suspension, a liquid medicine for internal use, an emulsion, a syrup, *etc.* In addition to a commonly used simple diluent such as water and liquid paraffin, various excipients such as a wetting agent, sweetener, aromatic, preservative, *etc.* may also be contained. Formulations for parenteral administration may include a sterilized aqueous solution, non-aqueous solution, suspension, emulsion, lyophilized formulation, and suppository. The non-aqueous solution or suspension may contain propylene glycol, polyethylene glycol, a vegetable oil such as olive oil, an injectable ester such as ethyl oleate, *etc.* Witepsol, macrogol, tween 61, cocoa butter, laurin butter, glycerogelatin, *etc.* may be used as a base of the suppository.

The pharmaceutical composition of the present disclosure may be administered in a pharmaceutically effective amount. As used herein, the term "pharmaceutically effective amount" refers to an amount sufficient for the treatment of diseases at a reasonable benefit/risk ratio applicable to a medical treatment, and the level of the effective dose may be determined from factors including severity of illness, drug activity, age, body weight, health conditions, drug sensitivity of a subject, administration time, administration route and dissolution rate, length of treatment of the pharmaceutical composition of the present disclosure, drug(s) used in combination with or simultaneously with the pharmaceutical composition of the present disclosure, and other factors well known in the medical field. The pharmaceutical composition of the present disclosure may be administered as an individual drug or in combination with other drug(s), and also sequentially or simultaneously with the conventional drug(s). Additionally, the pharmaceutical composition of the present disclosure may be administered as a single dose or in multiple divided doses. It is important that the least amount which can achieve the maximum effect without any side effects be administered in consideration of all of the factors described above.

It is preferable that the administration dose of the pharmaceutical composition of the present disclosure be, for example, 1.0×10⁹ CFU per day to a mammal including humans. The administration frequency may be, but is not particularly limited to, once daily, or divided into several doses. The administration dose does not limit the scope of the present disclosure in any aspect.

As another exemplary embodiment, the present disclosure provides a method for preventing or treating a degenerative brain disease comprising administering a pharmaceutically acceptable amount of the pharmaceutical composition into a subject having a risk of developing a degenerative disease or having a degenerative brain disease.

As previously described, at least one selected from the group consisting of the intestinal microorganism *Akkermansia muciniphila* strain, endoplasmic reticulum derived therefrom, culture thereof, concentrate of the culture, dry matter of the culture, and extract of the culture provided in the present disclosure can be used as an active ingredient of the pharmaceutical composition for preventing or treating a degenerative brain disease, and accordingly, the composition can be used in the prevention or treatment of a degenerative brain disease.

As used herein, the term "subject" includes without limitation all mammals including rats, livestock, and humans, having a risk of developing a degenerative disease or having a degenerative brain disease.

With respect to the method for treating a degenerative brain disease, the pharmaceutical composition can be administered via any conventional general route as long as it reaches target tissues. The pharmaceutical composition of the present disclosure can be administered orally, intrarectally, *etc.,* but is not limited thereto. In some cases, the pharmaceutical composition can be administered via other routes according to purposes. However, when orally administered, the intestinal microorganism *Akkermansia muciniphila* strain may be denatured due to stomach acid. Accordingly, compositions for oral administration should be coated with an active agent or formulated so as to protect from decomposition. Further, the composition can be administered by any device which enables the active substance to move to a target cell.

In addition, the present disclosure provides a health functional food composition for preventing or improving a degenerative brain disease, comprising as an active ingredient at least one selected from the group consisting of the strain, an endoplasmic reticulum derived therefrom, a culture thereof, a concentrate of the culture, a dry matter of the culture, and an extract of the culture.

The strain is the same as previously described, and can be added to a health functional food for the purpose of preventing or improving a degenerative brain disease.

The health functional food composition according to an exemplary embodiment of the present disclosure for preventing or improving degenerative brain diseases may further comprise at least one selected from nutrients, vitamins, electrolytes, flavoring agents, coloring agents, pectic acids and salts thereof, alginic acids and salts thereof, organic acids, protective colloid thickening agents, pH-adjusting agents, stabilizers, preservatives, glycerin, alcohols, carbonating agents used in carbonated drinks, *etc.*

When the strain, culture thereof, *etc.* are used as a health functional food composition, the strain or culture thereof can be added as it is or can be used with other foods or food ingredients, and can be used appropriately according to conventional methods. Amounts of active ingredients being mixed can be appropriately determined according to purpose of use (prevention, health, or therapeutic treatment).

The food (or health functional food) of the present disclosure may further comprise a conventionally added and sitologically acceptable ingredient. For example, when manufactured in the form of a drink, the food may further comprise, in addition to the strain of the present disclosure, at least one ingredient among citric acid, high fructose corn syrup, sugar, glucose, acetic acid, malic acid, fruit juice, *etc.*

The amount of the composition which can be included as the active ingredient in the food (or health functional food) according to the present disclosure may be appropriately determined depending on the age, gender, body weight, physical condition, and symptoms of the disease of a patient in need of the food for preventing or improving a degenerative brain disease. It is preferable that the composition be included in a daily amount of 0.01 g to 10.0 g per adult, and the effect of preventing or improving the degenerative brain disease can be obtained by ingesting a food having such amount.

Further, the present disclosure provides a method for producing a microorganism formulation for preventing or treating a degenerative brain disease comprising culturing the strain.

The method for culturing the strain of the present disclosure may be one conventionally used in the art.

The microorganism formulation of the present disclosure for preventing or treating a degenerative brain disease can be prepared using the *Akkermansia muciniphila* strain as an active ingredient. The microorganism formulation according to the present disclosure for preventing or treating a degenerative brain disease can be prepared in the form of solution, powder, suspension, dispersion, emulsion, oil-based dispersion, paste, dust, propellant, or granule, but is not limited thereto.

Still another aspect of the present disclosure provides a feed additive composition for preventing or improving a degenerative brain disease, comprising as an active ingredient at least one selected from the group consisting of an *Akkermansia muciniphila* strain cultured in a mucin-free medium, an endoplasmic reticulum derived therefrom, a culture thereof, a concentrate of the culture, a dry matter of the culture, and an extract of the culture.

The strain is the same as previously described, and can be added as a feed additive composition for the purpose of preventing or improving a degenerative brain disease. The feed additive composition of the present disclosure corresponds to a supplementary feed according to the Control of Livestock and Fish Feed Act.

The feed additive composition of the present disclosure can be added to a feed, wherein the term "feed" is any appropriate natural or artificial diet, single meal, *etc.* for animals to eat, ingest, and digest, or an element of the single meal. The type of the feed is not particularly limited, but any feed conventionally used in the technical field of known art can be used. Unlimited examples of the feed include vegetable feeds, such as cereals, nuts, food processed by-products, millet, fibers, pharmaceutical by-products, fats, starches, gourds, or grain by-products, *etc.,* and animal feed, such as protein, inorganic substances, fats, minerals, single cell proteins, animal planktons, or food, *etc.*

To achieve the purpose, the present disclosure provides a pharmaceutical composition for preventing or improving a metabolic disease, comprising as an active ingredient at least one selected from the group consisting of an *Akkermansia muciniphila* strain cultured in a mucin-free medium, an endoplasmic reticulum derived therefrom, a culture thereof, a concentrate of the culture, a dry matter of the culture, and an extract of the culture.

The *Akkermansia muciniphila* strain of the present disclosure was furnished from the American Type Culture Collection (ATCC) to use in the present disclosure (Accession No. ATCC BAA-835, identical to the strain of German Collection of Microorganisms and Cell Cultures Accession No. DSMZ 22959).

In general, any mucin-free medium can be used to culture the *Akkermansia muciniphila* strain of the present disclosure. Any medium (preferably CM medium, BHI medium, BTTM medium, *etc*.) in which an additive composition of the culture medium conventionally used for the culture of *Akkermansia muciniphila* strain can be used as long as it does not contain mucin.

In the pharmaceutical composition according to an exemplary embodiment for preventing or treating a metabolic disease, the metabolic disease can be one selected from the group consisting of hyperlipidemia, fatty liver, obesity, diabetes, arteriosclerosis, and hypertension, but is not limited thereto.

The pharmaceutical composition of the present disclosure for preventing or treating a metabolic disease may contain 0.02 wt% to 80 wt%, preferably 0.02 wt% to 50 wt% of dried powder or culture of the strain based on the total weight of the composition.

The composition of the present disclosure containing the dried powder or culture of the strain may further comprise a carrier, an excipient, or a diluent conventionally used in the preparation of a pharmaceutical composition, and the details in this regard are described hereinbelow.

The pharmaceutical administration of the dried powder or culture of the strain can be performed not only independently or as a conjugate with other pharmaceutically active compounds, but also as an appropriate combination, and the details in this regard are described hereinbelow.

An appropriate administration amount of the dried powder or culture of the strain may be appropriately determined by a person skilled in the art, although it may vary depending on the body weight and physical condition of a patient, severity of disease, dosage form, and administration route and period. For a preferred effect, however, it is preferable that the dried powder or culture of the strain be included in a daily amount of 0.0001 mg/kg to 100 mg/kg, preferably 0.001 mg/kg to 100 mg/kg.

The administration may be performed once daily, or divided into several doses. The administration dose does not limit the scope of the present disclosure in any aspect.

The dried powder or culture of the strain of the present disclosure can be administered to mammals including rats, mice, livestock, humans, *etc.* via various routes. All administration methods can be predicted; for example, oral or rectal administration, or intravenous, intramuscular, subcutaneous, or intra-endometrial injection.

In addition, the present disclosure provides a health functional food composition for preventing or improving a metabolic disease, comprising as an active ingredient at least one selected from the group consisting of an *Akkermansia muciniphila* strain cultured in a mucin-free medium, an endoplasmic reticulum derived therefrom, a culture thereof, a concentrate of the culture, a dry matter of the culture, and an extract of the culture.

In the health functional food composition according to an exemplary embodiment for preventing or improving metabolic diseases, the metabolic diseases may be selected from the group consisting of hyperlipidemia, fatty liver, obesity, diabetes, arteriosclerosis, and hypertension, but are not limited thereto.

The health functional food composition according to an exemplary embodiment of the present disclosure for preventing or improving metabolic diseases may further comprise at least one selected from nutrients, vitamins, electrolytes, flavoring agents, coloring agents, pectic acids and salts thereof, alginic acids and salts thereof, organic acids, protective colloid thickening agents, pH-adjusting agents, stabilizers, preservatives, glycerin, alcohols, carbonating agents used in carbonated drinks, *etc.*

The strain can be added to a health functional food for the purpose of preventing or improving metabolic diseases. When the strain, culture thereof, *etc.* are used as a health functional food composition, the strain or culture thereof can be added as it is or can be used with other foods or food ingredients, and can be used appropriately according to conventional methods. Amounts of active ingredients being mixed can be appropriately determined according to purpose of use (prevention, health, or therapeutic treatment).

The food (or health functional food) of the present disclosure may further comprise a conventionally added and sitologically acceptable ingredient. For example, when manufactured in the form of a drink, the food may further comprise, in addition to the strain of the present disclosure, at least one ingredient among citric acid, high fructose corn syrup, sugar, glucose, acetic acid, malic acid, fruit juice, *etc.*

The amount of the composition which can be included as the active ingredient in the food (or health functional food) according to the present disclosure may be appropriately determined depending on the age, gender, body weight, physical condition, and symptoms of the disease of a patient in need of the food for preventing or improving a metabolic disease. It is preferable that the composition be included in a daily amount of 0.01 g to 10.0 g per adult, and the effect of preventing or improving the metabolic disease can be obtained by ingesting a food having such amount.

Further the present disclosure provides a feed additive composition for preventing or improving a metabolic disease, comprising as an active ingredient at least one selected from the group consisting of an *Akkermansia muciniphila* strain cultured in a mucin-free medium, an endoplasmic reticulum derived therefrom, a culture thereof, a concentrate of the culture, a dry matter of the culture, and an extract of the culture.

The strain is the same as previously described, and can be added as a feed additive composition for the purpose of preventing or improving a metabolic disease. The feed additive composition of the present disclosure corresponds to a supplementary feed according to the Control of Livestock and Fish Feed Act.

The feed additive composition of the present disclosure can be added to a feed, wherein the term "feed" is any appropriate natural or artificial diet, single meal, *etc.* for animals to eat, ingest, and digest, or an element of the single meal. The type of the feed is not particularly limited, but any feed conventionally used in the technical field of known art can be used. Unlimited examples of the feed include vegetable feeds, such as cereals, nuts, food processed by-products, millet, fibers, pharmaceutical by-products, fats, starches, gourds, or grain by-products, *etc.,* and animal feed, such as protein, inorganic substances, fats, minerals, single cell proteins, animal planktons, or food, *etc.*

Further, the present disclosure provides a method for producing a microorganism formulation for preventing or treating a metabolic disease comprising culturing the strain.

The method for culturing the strain of the present disclosure may be one conventionally used in the art.

The microorganism formulation of the present disclosure for preventing or treating a metabolic disease can be prepared using the *Akkermansia muciniphila* strain cultured in a mucin-free medium as an active ingredient. The microorganism formulation according to the present disclosure for preventing or treating a metabolic disease can be prepared in the form of a solution, powder, suspension, dispersion, emulsion, oil-based dispersion, paste, dust, propellant, or granule, but is not limited thereto.

Hereinbelow, the constitution and effect of the present disclosure will be described in detail with accompanying exemplary embodiments. However, the exemplary embodiments disclosed herein are only for illustrative purposes and should not be construed as limiting the scope of the present disclosure.

### Example 1. Experiment on in vivo efficacy of Akkermansia muciniphila (AK) on Alzheimer's disease, dementia and cognitive function of brain

### 1-1. Experiment using a mouse model having overexpressed Alzheimer's disease genes

Mice models having Alzheimer's disease induced by the overexpression of APP and PSEN1 genes (Alzheimer's disease-related genes) was purchased from The Jackson Laboratory (B6C3-Tg(APPswe/PSEN1dE9)85DboJ, JAX, 004462). The experimental animal group was divided into a group of 5-month old normal mice (Non-Tg) having no overexpressed APP/PSEN1 (n=6) and a group of mice having Alzheimer's disease overexpressing APP/PSEN1 (n=15). The group of mice having Alzheimer's disease was further divided into a control group administered with 25% glycerol /PBS and an experimental group orally administered 2.0×10⁸ CFU of the *Akkermansia muciniphila* strain every day. The numbers of the mice in the control and experimental groups of Alzheimer's disease overexpressing the APP/PSEN1 were 9 and 6, respectively, where the control group was orally administered with 25% glycerol/PBS and the experimental group was orally administered with the *Akkermansia muciniphila* strain every day for 10 weeks. The *Akkermansia muciniphila* strain is an intestinal microorganism *Akkermansia muciniphila* (AK; American Type Culture Collection (ATCC) Accession No. ATCC BAA-835, identical to the strain of German Collection of Microorganisms and Cell Cultures Accession No. DSMZ 22959) which is already known as a standard strain, and was furnished from ATCC to use in the present disclosure.

### 1-2. Experiment using a mouse model having LPS-induced cognitive impairment

8-week-old male C57BL/6 mice were used: the control group was administered with 25% glycerol/PBS and the experimental group was daily orally administered with 2.0×10⁸ CFU of the *Akkermansia muciniphila* strain for 1 week. 250 µg/kg lipopolysaccharide (LPS) was further administered to both groups for 1 week intraperitoneally to induce a mouse model having cognitive function damage and Alzheimer-type dementia, and the effect of administration of the *Akkermansia muciniphila* strain was analyzed.

### 1-3. Recognition memory analysis

1-3-1. Y-maze test: Y-maze test was performed to measure the effect of the *Akkermansia muciniphila* strain of increasing spatial perception and memory. The alternation of the mouse was evaluated by measuring the entering number and order of the mouse in the pathways in the Y-shaped maze.
1-3-2. Novel Object Recognition Test (NORT): To measure the effect of the *Akkermansia muciniphila* strain of increasing cognitive ability and memory, the mouse was allowed for 10 minutes to explore the two identical cylindrical wood block placed on both side of a box. After 24 hours, the mouse was subjected to another object (novel object; square pillar-shaped) next to the cylindrical block (familiar object) and its activity was observed.

The time spent exploring the cylindrical block (familiar object) and that exploring the square pillar-shaped block (novel object) were measured with respect to the total time exploring both objects.

### 1-4. Effect at the activated microneuroglial cell level

To measure the anti-inflammatory response of the *Akkermansia muciniphila* strain in the brain, a brain tissue slice from control/control, control/LPS, and *Akkermansia muciniphila*/LPS-administered group was immunostained using an ionized calcium-binding adapter molecule 1 (Iba-1) antibody capable of specifically bind to activated microneuroglial cells. The level of the activated microneuroglial cells in the cerebral cortex of the control and experimental mice was measured to compare (Fig. 4). The control/control refers to a group with no LPS and AK administration, and control/LPS refers to a group administered with vehicle and LPS administration, while AK/LPS refers to a group administered with LPS and the *Akkermansia muciniphila* strain. Fig. 4A shows photos of the results of immunostaining of the microneuroglial cells activated in the brain of mouse, and Fig. 4B shows a graph of the result of comparison of change rate of immunostaining of the activated microneuroglial cells according to the administration of the *Akkermansia muciniphila* strain into the brain of LPS-administered model mouse. As shown in Figs. 4A and 4B, the level of the microneuroglial cells activated in the cerebral cortex is significantly reduced upon the *Akkermansia muciniphila* strain administration (**p* < 0.05).

### 1-5. Observation result

As a result of conducting the Y-maze text, the Alzheimer's disease mouse having overexpressed mutated APP/PSEN1 gene was observed to have low spatial perception and memory compared to normal mouse (non-tg). Further, the group of mice having prolonged administration of the *Akkermansia muciniphila* strain statistically showed an significant increase in their alternation (Student's t-test, **p*<0.05) (Fig. 1). Meanwhile, the mouse having Alzheimer's disease, in which the mutated gene of APP/PSEN1 is overexpressed, was shown to lack the novel object recognition memory; however, the group of mice administered with the *Akkermansia muciniphila* strain for a prolonged time exhibited remarkably increased novel object recognition memory (Student's *t*-test, **p*<0.05) (Fig. 2).

In the NORT, the control group having impaired cognitive functions by LPS administration showed remarkably decreased time for exploring novel object whereas the experimental group administered with the *Akkermansia muciniphila* strain together with LPS showed significantly increased time for exploring novel object (Student's *t*-test, **p*<0.05) (Fig. 3). Hyperactivation of brain inflammatory response due to LPS administration was also shown to remarkably reduce by the administration of the *Akkermansia muciniphila* strain (Student's *t*-test, **p*<0.05) (Fig. 4).

Based on such experimental results, the *Akkermansia muciniphila* strain was confirmed to have efficacy of effectively inhibiting cognition and memory damages in mice models having LPS-induced Alzheimer's disease related to overexpressed APP//PSEN1 mutation.

### Example 2. In vivo efficacy of Akkermansia muciniphila (AK) on Parkinson's diseases

### 2-1. Animal model and administration of Akkermansia muciniphila strain

8-week-old male C57BL/6 mice were used: the control group was administered with 25% glycerol/PBS and the experimental group was daily orally administered with 2.0×10⁸ CFU of the *Akkermansia muciniphila* strain for 1 week. 6-Hydroxydopamine (6-OHDA) was further added directly to the left corpus striatum to induce dopaminergic cell loss so as to manufacture an animal model having Parkinson's disease. The effect of administration of the *Akkermansia muciniphila* strain was analyzed.

### 2-2. Analysis of movement control dysfunction induced by Parkinson's disease

To ethologically analyze movement control dysfunction caused by Parkinson's disease according to the administration of the *Akkermansia muciniphila* strain, mice were subjected to D-amphetamine-induced rotation test to measure asymmetrical dyskinesia.

### 2-3. Observation result

Statistically, the *Akkermansia muciniphila* strain-administered group showed significant decrease in the rotational movements of Parkinson's disease-induced animals at all times (Student's *t*-test, **p*<0.05) (Figs. 5 and 6). Accordingly, it was confirmed based on such results that the *Akkermansia muciniphila* strain has prophylactic and therapeutic effects on Parkinson's disease.

### Example 3. Analysis of hyperlipidemia-improving effect of Akkermansia muciniphila (AK) strain when orally administered to an animal model having hyperlipidemia induced by high-fat diet

8-week-old male C57BL/6 mice, fed with high-fat feed for 6 weeks to induce alimentary obesity, were grouped in fives and kept feeding with high-fat feed, while the control group was administered with vehicle (25% glycerol/PBS) and the experimental groups were administered with 2.0×10⁸ CFU AK(+) proliferated in a mucin-containing medium and 1.0×10⁷ CFU AK(-) proliferated in a mucin-free medium, respectively, once daily for 5 weeks. After 5 weeks, the mice were fasted for 16 hours, followed by collecting blood thereof to measure the concentrations of fasting serum cholesterol and triglyceride to compare and analyze.

Plasma was separated from the blood collected from each animal after 16 hours of fasting, and blood cholesterol and triglyceride concentrations were measured to compare and analyze using a hematology chemistry analyzer. As a result, the control group showed fasting blood cholesterol and triglyceride concentrations of 191±12 mg/dL and 198±12 mg/dL, respectively, whereas the AK(+) strain-administered experimental group showed the concentrations of 183±6 mg/dL and 164±16 mg/dL and AK(-) strain-administered experimental group showed 156±9 mg/dL and 145±6 mg/dL of fasting blood cholesterol and triglyceride concentrations, indicating that AK strain administration serves to reduced blood cholesterol and triglyceride concentrations compared to the control group. The experimental group administered with the AK(-) strain showed significant decreases in the blood cholesterol and triglycerides compared to the control group and a stronger effect of ameliorating hyperlipidemia (*p*<0.01, Student's *t*-test) (Fig. 7).

Based on such results, the AK(-) strain cultured in a mucin-free medium showed stronger effect on amelioration of hyperlipidemia compared to the AK(+) strain cultured in a mucin-containing medium (Fig. 7).

### Example 4. Analysis of fatty liver-improving effect of Akkermansia muciniphila (AK) strain when orally administered to an animal model having hyperlipidemia induced by high-fat diet and alcohol

8-week-old male C57BL/6 mice, fed with high-fat feed for 6 weeks to induce alimentary obesity, were grouped in fives and kept feeding with high-fat feed, while the control group was administered with vehicle (25% glycerol/PBS) and the experimental groups were administered with 2.0×10⁸ CFU AK(+) proliferated in a mucin-containing medium and 1.0×10⁷ CFU AK(-) proliferated in a mucin-free medium, respectively, once daily for 5 weeks. After 5 weeks, the mice were fasted for 16 hours, and a liver tissue was obtained to prepare a cryostat section. Oil red-O (ORD) staining was then performed to histologically observe and compare the formation of fatty liver. Part of the liver tissue was used for a kit for measuring triglycerides to directly measure and comparatively analyze the concentration of the triglycerides present in the liver tissue.

As a result of the fatty liver analysis, it was found that there was no particular difference in the fatty liver stained with red color in the AK(+) strain-administered group compared to the control group. In the AK(-) strain-administered experimental group, however, significantly reduced formation of fatty liver was observed compared to the control group. Additionally, in the analysis of the triglyceride content in the liver, only the group administered with the AK(-) strain showed a significant reduction compared to the control group (*p*<0.01, Student's *t*-test) (Fig. 8).

Based on such results, the group administered with the AK(+) strain cultured in a mucin-containing medium did not exhibit fatty liver inhibition whereas the group administered with the AK(-) strain cultured in a mucin-free medium showed an effect of improving fatty liver induced by high-fat diet (Fig. 8).

### Example 5. Analysis of obesity-improving effect of Akkermansia muciniphila (AK) strain when orally administered to an animal model having obesity induced by high-fat diet

8-week-old male C57BL/6 mice, fed with high-fat feed for 6 weeks to induce alimentary obesity, were grouped in fives and kept feeding with high-fat feed, while the control group was administered with vehicle (25% glycerol/PBS) and the experimental groups were administered with 2.0×10⁸ CFU AK(+) proliferated in a mucin-containing medium and 1.0×10⁷ CFU AK(-) proliferated in a mucin-free medium, respectively, once daily for 4 weeks. After 0 and 4 weeks of administration, the weights of the mice were measured to calculate an individual body weight increase rate and compared with each other.

Both experimental groups administered with AK(+) strain cultured in a mucin-containing medium and AK(-) strain cultured in a mucin-free medium were observed to show 115±5.9% and 109±2.0% of the body weight increase rate, about 5% and 10% lower rate compared to the control group (119±1.4%) (*p*<0.01, Student's *t*-test) (Fig. 9).

Based on such results, the AK strains have an effect of inhibiting body weight increase; in particular, the AK(-) strain cultured in a mucin-free medium exhibited more excellent efficacy on inhibiting obesity induced by high-fat diet compared to the AK(+) strain cultured in a mucin-containing medium (Fig. 9).

### Example 6. Analysis of the effect of Akkermansia muciniphila (AK) strain of dropping blood sugar when orally administered to an animal model having hyperglycemia induced by high-fat diet

8-week-old male C57BL/6 mice, fed with high-fat feed for 6 weeks to induce alimentary obesity, were grouped in fives and kept feeding with high-fat feed, while the control group was administered with vehicle (25% glycerol/PBS) and the experimental groups were administered with 2.0×10⁸ CFU AK(+) proliferated in a mucin-containing medium and 1.0×10⁷ CFU AK(-) proliferated in a mucin-free medium, respectively, once daily for 4 weeks. After 4 weeks of administration, non-fasting blood glucose with unlimited diet and fasting blood glucose after 16 hours of fasting were directly measured from the blood using a glucometer and comparatively analyzed.

After 4 weeks of AK strain-administration, the fasting blood glucose levels of the conditions of having no limitation on diet and fasting for 16 hours appeared to be 206±6 mg/dL and 105±6 mg/dL, respectively (control groups), 206±6 mg/dL and 113±8 mg/dL (the AK(+) strain cultured in a mucin-containing medium), and 166±8 mg/dL and 88±3 mg/dL (the AK(-) strain cultured in a mucin-free medium). That is, compared to the control group, the AK(+)-administered group did not show significant difference whereas the AK(-)-administered group showed significant decreases in both conditions of having no limitation on diet and fasting for 16 hours. This indicates that the AK(-)-administered group has a greater effect on dropping the blood glucose compared to the AK(+)-administered group (*p*<0.01, Student's *t*-test) (Fig. 10).

Based on such results, the AK(-) strain cultured in a mucin-free medium was shown to effectively inhibit the hyperglycemia induced by high-fat diet, which was not observed in the AK(+) strain cultured in a mucin-containing medium (Fig. 10).

### Example 7. Analysis of glucose intolerance impairment-ameliorating effect of Akkermansia muciniphila (AK) strain when orally administered to an animal model having glucose intolerance impairment induced by high-fat diet

8-week-old male C57BL/6 mice, fed with high-fat feed for 6 weeks to induce alimentary obesity, were grouped in fives and kept feeding with high-fat feed, while the control group was administered with vehicle (25% glycerol/PBS) and the experimental groups were administered with 2.0×10⁸ CFU AK(+) proliferated in a mucin-containing medium and 1.0×10⁷ CFU AK(-) proliferated in a mucin-free medium, respectively, once daily for 4 weeks. After 4 weeks of administration, the mice were fasted for 16 hours and peritoneally administered with 2 g/kg glucose to measure blood glucose levels after 0, 30, 60, 90, and 120 minutes. Using glucose tolerance test, the glucose intolerance impairment-ameliorating effect was analyzed.

To verify the improvement of glucose intolerance impairment induced by high-fat diet, the AK(+) and AK(-) strains were administered for 4 weeks and glucose was peritoneally administered to measure blood glucose at every hour and compare them. As a result, the control group showed an increase up to 300 mg/dL in 30 minutes after the glucose administration and gradually decreased until it reached 120 minutes, whereas the AK(+)-administered group showed lower glucose levels at 30 minutes and 60 minutes compared to the control group, and the AK(-)-administered group showed significantly decreased blood glucose at 30, 60, 90, and 120 minutes compared to the control group (*p*<0.01, Student's *t*-test) (Fig. 11A). Further, Area Under Curve (AUC), a bar graph represented by measuring the area of the region between the X axis and the line graph presenting blood glucose level measured according to time upon glucose administration was measured for the vehicle and the AK strains-administered groups, and as a result, it was found that the AK(-) strain-administered experimental group showed significant reduction compared with the control group, thereby indicating its strong improvement effect of impaired glucose tolerance (Fig. 11B).

Based on such results, the AK(-) strain cultured in a mucin-free medium showed more excellent effect of ameliorating glucose tolerance impairment induced by high=-fat diet compared to the AK(+) strain cultured in a mucin-containing medium (Fig. 11).

### Example 8. Analysis of the effect of Akkermansia muciniphila (AK) strain of ameliorating reduced sensitivity to insulin when orally administered to an animal model having reduced sensitivity to insulin induced by high-fat diet

8-week-old male C57BL/6 mice, fed with high-fat feed for 6 weeks to induce alimentary obesity, were grouped in fives and kept feeding with high-fat feed, while the control group was administered with vehicle (25% glycerol/PBS) and the experimental groups were administered with 2.0×10⁸ CFU AK(+) proliferated in a mucin-containing medium and 1.0×10⁷ CFU AK(-) proliferated in a mucin-free medium, respectively, once daily for 4 weeks. After 4 weeks of administration, the mice were subjected to insulin tolerance test; they were fasted for 4 hours, and injected with insulin, followed by measuring blood glucose at 0, 30, 60, 90, and 120 minutes to comparatively analyze the amelioration of reduced sensitivity to insulin. In addition, after 4 weeks of administration, they were fasted for 16 hours to measure their fasting blood glucose and insulin concentrations. Based on the measurements, Homeostasis Model Assessment of insulin resistance index (HOMA-IR; [Fasting insulin (µIU/mL) × fasting plasma glucose (mmol/L)] / 22.5), generally used as an index for insulin resistance, was calculated.

To verify amelioration of reduced sensitivity to insulin induced by high-fat diet, the AK(+) and AK(-) strains were administered for 4 weeks. After 4 hours of fasting, insulin was administered and the blood glucose levels were measured according to time. As a result, the blood glucose level of the control group at 60 minutes decreased to about 60% of that at 0 minutes of the insulin administration, and recovered up to about 85% at 120 minutes. The experimental groups administered with the AK(+) and AK(-) strains showed significantly reduced blood glucose level at 90 minutes after the administration, *i.e.,* about 40%; however, the AK(+)-strain administered group recovered its blood glucose level up to 59%, and AK(-)-administered group recovered its blood glucose level up to 42% at 120 minutes (*p*<0.01, Student's *t*-test) (Fig. 12). In comparison of the AK(+) and AK(-) strains, the group administered with the AK(-) strain showed stronger and longer effect of ameliorating reduced sensitivity to insulin (Fig. 12). Further, the control group showed the highest fasting blood insulin concentration (1.1±0.1 ng/mLl), whereas the experimental groups administered with the AK(+) and AK(-) strains showed 0.7±0.1 ng/mL and 0.6±0.1 ng/mL of fasting blood insulin concentrations, indicating significant reduction of blood insulin concentrations compared to the control group (Fig. 13). In addition, the AK strain-administered groups showed lower HOMA-IR values than the control group (9.8±1.9); in particular, the experimental group administered with the AK(-) strain showed an even lower HOMA-IR value of 4.9±0.7 than that administered with the AK(+) strain having the HOMA-IR value of 6.3±1.6. This demonstrates that the administration of the AK strain in a mouse model having obesity and hyperglycemia induced by high-fat diet would improve resistance to insulin, and that the AK(-) strain cultured in a mucin-free medium showed stronger effects (Fig. 14).

Based on such results, the AK(-) strain cultured in a mucin-free medium showed stronger effect on amelioration of reduction of sensitivity to insulin induced by high-fat diet compared to the AK(+) strain cultured in a mucin-containing medium (Figs. 12 to 14).

### Example 9. Analysis of distribution of white adipose tissue by size and of pathological changes according to oral administration of Akkermansia muciniphila (AK) strain in an animal model having obesity induced by high-fat diet

8-week-old male C57BL/6 mice, fed with high-fat feed for 6 weeks to induce alimentary obesity, were grouped in fives and kept feeding with high-fat feed, while the control group was administered with vehicle (25% glycerol/PBS) and the experimental groups were administered with 2.0×10⁸ CFU AK(+) proliferated in a mucin-containing medium and 1.0×10⁷ CFU AK(-) proliferated in a mucin-free medium, respectively, once daily for 4 weeks. After 5 weeks of administration, they were fasted for 16 hours, and white adipose tissue (abdominal fat and subcutaneous fat) and brown adipose tissue were collected to conduct H & E staining and observe the size of adipocytes. The brown adipose tissue was much smaller in size than the white adipose tissue and thus could not be applied by the image analysis program; however, in the case of white adipose tissue, the size of each adipocyte was calculated by utilizing the image analysis program, and distribution diagram of adipocytes according to size was calculated based on the calculation. As a result, the adipose tissue of the AK(-) strain-administered experimental group showed significantly low distribution of abdominal fat and subcutaneous fat in the size of ≥2500 µm² whereas they showed high distribution for the adipose tissues in the smaller size of ≤2500 µm² (Figs. 15 and 16). Further, the brown adipose tissue was observed through H&E staining, and it appeared that most of the adipose tissue of the no strain-treated experimental group HFD having high-fat diet were whitened and became larger in size, whereas the experimental groups administered with the AK strains showed less whitening. In particular, the experimental group administered with the AK(-) strain cultured in a mucin-free medium (HFD+AK(-)) showed more brown adipocytes maintained compared to the experimental group administered with the AK(+) strain cultured in a mucin-containing medium (HFD+AK(+)) (Fig. 17).

Based on such results, the AK(-) strain cultured in a mucin-free medium effectively inhibited proliferation of adipocytes induced by high-fat diet.

## Claims

1. An *Akkermansia muciniphila* strain having a prophylactic or therapeutic effect on a degenerative brain disease.

2. A pharmaceutical composition for preventing or treating a degenerative brain disease, comprising as an active ingredient at least one selected from the group consisting of the strain according to claim 1, an endoplasmic reticulum derived therefrom, a culture thereof, a concentrate of the culture, a dry matter of the culture, and an extract of the culture.

3. The pharmaceutical composition according to claim 2, wherein the degenerative brain disease is Alzheimer's disease, Parkinson's disease, mild cognitive impairment, meningitis, stroke, dementia, Huntington's disease, or Creutzfeldt-Jakob disease.

4. The pharmaceutical composition according to claim 2, wherein the composition has an inhibition effect on movement disorders due to dopaminergic neuron death.

5. The pharmaceutical composition according to claim 2, wherein the composition has an inhibition effect on cerebral inflammatory responses.

6. The pharmaceutical composition according to claim 2, wherein the composition has an improvement effect on cognitive ability and memory.

7. A health functional food composition for preventing or improving a degenerative brain disease, comprising as an active ingredient at least one selected from the group consisting of the strain according to claim 1, an endoplasmic reticulum derived therefrom, a culture thereof, a concentrate of the culture, a dry matter of the culture, and an extract of the culture.

8. The health functional food composition according to claim 7, further comprising in addition to the active ingredient at least one selected from a nutrient, a vitamin, an electrolyte, a flavoring agent, a coloring agent, an enhancer, a pectic acid and a salt thereof, an alginic acid and a salt thereof, an organic acid, a protective colloid thickening agent, a pH-adjusting agent, a stabilizer, a preservative, glycerin, an alcohol, and a carbonating agent used for carbonated drinks.

9. A feed additive composition for preventing or improving a degenerative brain disease, comprising as an active ingredient at least one selected from the group consisting of the strain according to claim 1, an endoplasmic reticulum derived therefrom, a culture thereof, a concentrate of the culture, a dry matter of the culture, and an extract of the culture.

10. A pharmaceutical composition for preventing or treating a metabolic disease, comprising as an active ingredient at least one selected from the group consisting of an *Akkermansia muciniphila* strain cultured in a mucin-free medium, an endoplasmic reticulum derived therefrom, a culture thereof, a concentrate of the culture, a dry matter of the culture, and an extract of the culture.

11. The pharmaceutical composition according to claim 10, wherein the metabolic disease is one selected from the group consisting of hyperlipidemia, fatty liver, obesity, diabetes, arteriosclerosis, and hypertension.

12. The pharmaceutical composition according to claim 10, further comprising a carrier, an excipient, or a diluent, in addition to the active ingredient.

13. A health functional food composition for preventing or improving a metabolic disease, comprising as an active ingredient at least one selected from the group consisting of an *Akkermansia muciniphila* strain cultured in a mucin-free medium, an endoplasmic reticulum derived therefrom, a culture thereof, a concentrate of the culture, a dry matter of the culture, and an extract of the culture.

14. The health functional food composition according to claim 13, wherein the metabolic disease is one selected from the group consisting of hyperlipidemia, fatty liver, obesity, diabetes, arteriosclerosis, and hypertension.

15. The health functional food composition according to claim 13, further comprising in addition to the active ingredient at least one selected from a nutrient, a vitamin, an electrolyte, a flavoring agent, a coloring agent, an enhancer, a pectic acid and a salt thereof, an alginic acid and a salt thereof, an organic acid, a protective colloid thickening agent, a pH-adjusting agent, a stabilizer, a preservative, glycerin, an alcohol, and a carbonating agent used for carbonated drinks.

16. A feed additive composition for preventing or improving a metabolic disease, comprising as an active ingredient at least one selected from the group consisting of an *Akkermansia muciniphila* strain cultured in a mucin-free medium, an endoplasmic reticulum derived therefrom, a culture thereof, a concentrate of the culture, a dry matter of the culture, and an extract of the culture.
